# EUROPÄISCHE PATENTSCHRIFT

(11) **EP 1 593 654 B1**
(45) Veröffentlichungstag und Bekanntmachung des Hinweises auf die Patenterteilung: **24.04.2013**
(21) Anmeldenummer: 05006943.4
(22) Anmeldetag: 30.03.2005
(51) Int. Cl.: C02F 1/02, C02F 9/00, C07C 201/16

(54) **Verfahren zur Aufarbeitung von aromatische Nitroverbindungen enthaltenen Abwässern**
Process for treating waste waters containing aromatic nitro compounds
Procédé de traitement d'eaux usées contenant des composés nitro-aromatiques

(30) Priorität: 10.04.2004 DE 102004017628
(43) Veröffentlichungstag der Anmeldung: 09.11.2005
(73) Patentinhaber: Bayer MaterialScience AG, 51368 Leverkusen (DE)
(72) Erfinder: Knauf, Thomas, Dr., 41542 Dormagen (DE); von Gehlen, Franz-Ulrich, 47839 Krefeld (DE); Dohmen, Wolfgang, 47229 Duisburg (DE); Schmiedler, Jörg, 47228 Duisburg (DE)

(56) Entgegenhaltungen:
- EP-A- 0 005 203
- EP-A- 0 503 387
- EP-A- 1 132 347
- DE-A1- 19 815 844
- US-A- 3 844 914
- US-A- 4 925 565
- US-A- 5 221 440

## Beschreibung

Die Erfindung betrifft ein Verfahren zur Aufarbeitung von alkalischen Abwässern, die bei der Wäsche von rohem Nitrobenzol entstehen durch thermische Druckzersetzung (TDZ).

Diese alkalischen Abwässer enthalten üblicherweise neben Wasser noch Restmengen an Benzol und Nitrobenzol sowie Nitrohydroxyaromaten. Beispielsweise seien folgende Nitrohydroxyaromaten, die auch in Form ihrer wasserlöslichen Salze vorliegen können, genannt: Mono-, Di- und Trinitrophenole, Mono-, Di- und Trinitrokresole, Mono-, Di- und Trinitroresorcine, Mono-, Di-und Trixylenole. Als Salzbildner kommen alle Metalle in Betracht, die mit den Nitrohydroxyaromaten wasserlösliche Salze zu bilden vermögen. Bevorzugt seien die Alkalimetalle genannt, z.B. Lithium, Natrium, Kalium und Rubidium.

Das grundsätzliche Verfahren der TDZ zur Behandlung von aromatischen Nitroverbindungen enthaltenden Abwässern ist in folgenden Patenten beschrieben. In EP 0 005 203 A2 ist ein Verfahren beschrieben zur Aufarbeitung von Nitrohydroxyaromaten enthaltenden Abwässern, wobei die Abwässer unter Luft- und Sauerstoffausschluss bei einem Druck von 50 - 250 bar und mit einer Temperatur von 150 - 500°C behandelt werden.

In EP 0 503 387 A1 wurde ein ähnliches Verfahren beschrieben, das aber dadurch gekennzeichnet ist, dass das besagte alkalische Abwasser durch Salpetersäurezugabe und anschließende Behandlung in Temperaturenbereichen von 180 - 350°C und einem Druckbereich von 40 - 250 bar aufgearbeitet wird.

Beide Verfahren weisen jedoch erhebliche Nachteile auf.

EP 0 005 203 A2 beschreibt nicht die Entfernung von organischen Kohlenwasserstoffen wie Benzol oder Nitrobenzol, die in einem dem technischen Stand entsprechenden adiabaten Nitrierprozess anfallen. Die Reinigung des Abwassers gemäß der Lehre von EP 0 005 203 A2 ist daher unzureichend oder der Verbrauch an Natronlauge in der TDZ wird sehr hoch.

In EP 0 503 387 A1 gelingt nicht die vollständige Zersetzung von Nitrobenzol, so dass eine weitere Behandlung des Abwassers notwendig ist. Außerdem wird das im Abwasser enthaltene Nitrobenzol in der TDZ zersetzt und mindert daher die erzielte Ausbeute. Die nach der Lehre von EP 0 503 387 A1 erforderliche Anwesenheit von Salpetersäure in der TDZ treibt zudem die Prozesskosten in zweierlei Hinsicht in die Höhe: zum einen durch den Verbrauch an Salpetersäure und zum anderen durch die hohen Materialbelastungen und die damit verbunden hohen Investitionskosten für einen titanausgekleideten Rohrreaktor. Ein zusätzlicher, nicht beschriebener Nachteil ist auch die Notwendigkeit, das beschriebene alkalische Abwasser vor Zugabe der Salpetersäure neutralisieren zu müssen, was gegebenenfalls mit dem entsprechenden Äquivalent an Salpetersäure erfolgen kann.

Die Aufgabe der vorliegenden Erfindung war daher, ein einfaches und wirtschaftliches Verfahren zur Aufarbeitung von alkalischen Abwässern, die bei der Wäsche von durch adiabate Nitrierung von Benzol hergestelltem rohem Nitrobenzol entstehen, zur Verfügung zu stellen. Dabei soll das Verfahren durch niedrige Investitionskosten realisierbar sein (d.h., Verzicht auf mit Titan ausgekleideten Apparaten in der TDZ), geringen Verbrauch an NaOH aufweisen und gleichzeitig eine hohe Reinigungswirkung aufweisen.

Die Erfindung betrifft ein Verfahren zur Aufarbeitung von alkalischen Abwässern, die bei der Wäsche von rohem Nitrobenzol entstehen, wobei das rohe Nitrobenzol
a) durch adiabate Nitrierung von Benzol mit Nitriersäure hergestellt, und anschließend
b) in einer sauren Wäsche gewaschen, und anschließend
c) in einer alkalischen Wäsche gewaschen wird, wobei ein alkalisches Abwasser enthaltend Benzol in Konzentrationen von 100 bis 3000 ppm und Nitrobenzol in Konzentrationen von 1000 bis 10000 ppm erhalten wird,
   dadurch gekennzeichnet, dass
d) aus dem alkalischen Abwasser aus Schritt c) anschließend nicht gelöst vorliegendes Benzol und/oder Nitrobenzol abgeschieden wird, und
e) aus dem alkalischen Abwasser aus Schritt d) anschließend restliches Benzol und/oder Nitrobenzol durch Strippen entfernt wird, und
f) das alkalische Abwasser aus Schritt d) oder e) anschließend unter Ausschluss von Sauerstoff auf Temperaturen von 150 bis 500°C unter Überdruck erhitzt wird.

Die Nitrierung von Benzol zu Nitrobenzol in Schritt a) erfolgt dabei üblicherweise nach den Verfahren nach dem Stand der Technik, z.B. gemäß EP 043 6 443 A2.

Das in Schritt a) hergestellte rohe Nitrobenzol wird anschließend in einer sauren Wäsche in Schritt b) gewaschen. Bevorzugt wird dabei eine Säurekonzentration von 0,5 bis 2 Gew.-% Schwefelsäure bezogen auf die wässrige Phase eingestellt.

Das rohe Nitrobenzol wird anschließend in Schritt c) in einer alkalischen Wäsche gewaschen. Bevorzugt wird dabei ein pH-Wert von ≥ 9, besonders bevorzugt von 10 bis 14 eingestellt. Das dabei erhaltene alkalische Abwasser enthält Benzol in Konzentrationen von 100 bis 3000 ppm, vorzugsweise von 100 bis 1000 ppm und Nitrobenzol in Konzentrationen von 1000 bis 10000 ppm, vorzugsweise von 1200 bis 8000 ppm. Das Abwasser enthält ferner üblicherweise Nitrohydroxyaromaten in einer Konzentration von 2000 bis 25000 ppm. Das so erhaltene alkalische Abwasser wird dann in den Schritten d) bis f) weiter aufgearbeitet.

In Schritt d) wird aus dem alkalischen Abwasser noch enthaltenes, nicht gelöst vorliegendes Benzol und/oder Nitrobenzol abgetrennt. Das so abgetrennte Benzol und/oder Nitrobenzol wird dann bevorzugt wieder dem Nitrierprozess bzw. dem rohen Nitrobenzol zugeführt. Die Abtrennung des nicht gelöst vorliegenden Nitrobenzols kann dabei durch Abscheider, Absetzbehälter oder andere Phasentrennapparaturen erfolgen. Bevorzugt wird ein Absetzbehälter eingesetzt. Bevorzugt wird in Schritt d) ein alkalisches Abwasser erhalten, das Benzol in Konzentrationen von 100 bis 1000 pppm und Nitrobenzol in Konzentrationen von 1200 bis 3000 ppm enthält.

Aus dem aus Schritt d) erhaltenen alkalischen Abwasser wird Benzol und gegebenenfalls restliches Nitrobenzol durch Strippen entfernt. Die Strippung erfolgt dabei vorzugsweise in einer Strippkolonne durch Abstrippen der Restmengen an Benzol und Nitrobenzol mit Wasserdampf über Kopf. Die anfallenden, Benzol und Nitrobenzol enthaltenden Brüden werden dann bevorzugt in die alkalische Wäsche in Schritt c) zurückgeführt. Eine Fehlfunktion der Strippkolonne kann beispielsweise durch redundante Sicherheitseinrichtungen überwacht werden. Bevorzugt wird in Schritt e) ein alkalisches Abwasser erhalten, das Benzol nur noch in Konzentrationen von bis zu 10 ppm, und Nitrobenzol in Konzentrationen von bis zu 10 ppm enthält.

In Schritt f) wird das aus dem Schritt e) erhaltene alkalische Abwasser, das noch mit organischen Salzen der Nitrohydroxyaromaten beladen ist, unter Ausschluss von Sauerstoff auf Temperaturen von 150 bis 500°C, vorzugsweise von 250 bis 350°C, besonders bevorzugt 270 bis 290°C unter Überdruck erhitzt. Es ist auch möglich, die Abwässer unter Inertgasatmosphäre bzw. unter einem Inertgas-Vordruck von beispielsweise 0,1 bis 100 bar zu erhitzen. Als Inertgase sind z.B. Stickstoff und/oder Argon geeignet. Je nach Temperatur und gegebenenfalls Inertgas-Vordruck stellen sich beim Erhitzen der Abwässer bevorzugt absolute Drücke im Bereich von 50 bis 350 bar, besonders bevorzugt 50 bis 200 bar, ganz besonders bevorzugt 70 bis 130 bar, ein. Die Erhitzung des alkalischen Abwassers und thermische Druckzersetzung der organischen Bestandteile wie Benzol, Nitrobenzol und Nitrohydroxyaromaten erfolgt dabei üblicherweise für 5 bis 120 min, bevorzugt 15 bis 30 min.

Die Anlagenteile der TDZ können dabei beispielsweise aus dem vergleichsweise günstigen Stahl 1.4571 gefertigt werden. Eine Titanbeschichtung der abwasserberührten Teile ist nicht notwendig. Das so behandelte Abwasser durchläuft anschließend bevorzugt eine Sicherheitseinrichtung, in welcher der Gehalt an Phenolaten überprüft wird, und kann dann beispielsweise in eine biologische Kläranlage abgegeben werden.

Durch die Strippung in Schritt e) können mit dem erfindungsgemäßen Verfahren Benzol und Nitrobenzol auf Gehalte ≤ 2 ppm abgebaut werden. Mit dem erfindungsgemäßen Verfahren können auch die Nitrohydroxyaromaten in der TDZ auf Gehalte < 10 ppm, bevorzugt < 5 ppm abgebaut werden.

Bevorzugt wird das erfindungsgemäße Verfahren so betrieben, dass zwischen den Schritten d) bzw. e) und Schritt f) in einer Sicherheitseinrichtung die Konzentrationen an Aromaten in dem alkalischen Abwasser überprüft wird, um eine mögliche Explosionsgefahr in der TDZ auszuschließen. Dazu erfolgt bevorzugt in der alkalischen Wäsche in Schritt c) eine Trennschichtüberwachung, (Überwachung, dass die abgezogene wässrige Phase weitgehend frei von organischen Bestandteilen ist) in der Abtrennung von Benzol und Nitrobenzol in Schritt d) eine Trennschichtüberwachung sowie eine pH-Wert und/oder Dichteüberwachung und gegebenenfalls nach Schritt e) zusätzlich eine FID-Überwachung (Flammen-Ionisations-Detektor).

Den alkalischen Abwässern aus der Wäsche des rohen Nitrobenzols können auch Anilin und Aminohydroxyaromaten enthaltende Abwässer, die beispielsweise bei der Herstellung von Anilin anfallen, zugemischt werden. Diese Abwässer aus der Anilinherstellung enthalten dabei bevorzugt aliphatische und aromatische Kohlenwasserstoffe in Konzentrationen von 1 bis 10 ppm sowie Phenole und deren Salze in Konzentrationen von 200 bis 1500 ppm.

Das erfindungsgemäße Verfahren weist die folgenden Vorteile auf: da aus dem alkalischen Abwasser in Schritt d) Benzol und/oder Nitrobenzol und in Schritt e) noch restliches Benzol und/oder Nitrobenzol weitgehend entfernt werden, wird in der TDZ in Schritt f) weniger NaOH verbraucht. Entsprechend ist der Verbrauch an NaOH im Verfahren und der Verlust an Benzol und Nitrobenzol durch die Zersetzung in der TDZ minimiert. Durch den geringeren Einsatz an NaOH wird gleichzeitig erreicht, dass keine hochkorrosionsbeständigen Werkstoffe für die Anlagenteile der TDZ eingesetzt werden müssen. Da in der TDZ wegen der vorherigen Abtrennung von Benzol und Nitrobenzol insgesamt weniger organische Bestandteile abgebaut werden müssen, ergibt sich letztlich auch eine größere Kapazität der TDZ und bessere Reinheiten des so aufgearbeiteten Abwassers. Die nach dem erfindungsgemäßen Verfahren behandelten Abwässer können daher ohne Verdünnung direkt einer biologischen Kläranlage zugeführt werden.

### Beispiele

### Beispiel 1 (Vergleichsbeispiel EP 0 005 203 A2)

In einen mit Stickstoff gespülten 2-1-Autoklaven mit Rührer, Druck- und Temperaturmessung werden 1200 ml eines wässrig-alkalischen Abwassers aus einem adiabaten Nitrierprozess eingefüllt. Anschließend wird Stickstoff mit einem Druck von 30 bar aufgepresst. Dann wird auf 300°C aufgeheizt und 15 Minuten bei der Reaktionstemperatur von 300°C gehalten (TDZ). Der Druck steigt hierbei auf 114 bar. Nach dem Abkühlen wird das Abwasser entnommen und analysiert. In Tabelle 1 sind die Analysenwerte der Gehalte der verschiedenen organischen Substanzen vor und nach der thermischen Druckzersetzung (TDZ) aufgeführt.

**Tabelle 1**

| **Gehalt** | **vor der TDZ** | **nach der TDZ** |
|---|---|---|
| Na-Salze der Mono-, Di-, und Tri-nitrophenole | 3100 ppm | 2,6 ppm |
| Natriumhydroxid | 0,25 Gew.% | 0,12 Gew.-% |
| Natriumsulfat | 0,5 Gew.-% | 0,5 Gew.-% |
| Natriumnitrat | 1,8 Gew.-% | 2,1 Gew.% |
| Natriumnitrit | 0,04 Gew.-% | 0,05 Gew.-% |
| Natriumcarbonat | 0,05 Gew.-% | 0,2 Gew.-% |

### Beispiel 2 (Vergleichsbeispiel EP 0 503 387 A1)

Ein Abwasser mit der in Tabelle 2 angegebenen Zusammensetzung wird unter folgenden Reaktionsbedingungen in einem Rohrreaktor behandelt:
- Reaktionstemperatur: 280° bis 290°C
- Druck: 95 bar
- Salpetersäurezugabe: 1,5 Gew.-% bezogen auf das Gewicht des Abwassers
- Verweilzeit: 5 min.

**Tabelle 2**

| **Analyse des Abwassers** | **vor der Behandlung** | **nach der Behandlung** |
|---|---|---|
| TOC-Gehalt (TOC: total organic carbon) | 355 mg/l | 17 mg/l |
| Nitrobenzol | 2000 mg/l | 6 mg/l |
| 2,6-Dinitrophenol | 53 mg/l | < 100 ppb |
| 2,4-Dinitrophenol | 485 mg/l | < 100 ppb |
| Pikrinsäure | 117 mg/l | < 50 ppb |

### Beispiel 3 (Vergleichsbeispiel)

Das rohe Nitrobenzol aus der adiabaten Nitrierung von Benzol wird zunächst in einer sauren Wäsche gewaschen und anschließend unter Zugabe von Natronlauge (50%ig) in einem Rührwerksbehälter alkalisch gewaschen. Danach wird das Gemisch in einem nachgeschalteten ersten Trennbehälter aufgrund der Dichteunterschiede in eine organische Phase (Rohnitrobenzol) und eine wässrige Phase (alkalisches Abwasser, Ablauge) getrennt. Das alkalische Abwasser ist mit Nitrobenzol gesättigt und liegt in der in Tabelle 3 angegebenen Zusammensetzung vor. Das alkalische Abwasser wird anschließend in einen weiteren Trennbehälter gefördert. In diesem Trennbehälter setzt sich nicht gelöstes Nitrobenzol und Benzol am Boden des Trennbehälters ab und wird durch Phasentrennung abgetrennt. Das alkalische Abwasser aus diesem Trennbehälter wird mit Abwasser aus der Anilin-Aufarbeitung vermischt und zur weiteren Behandlung in eine thermische Druckzersetzung geleitet. In Tabelle 3 sind die Analysendaten der Zusammensetzung des alkalischen Abwassers vor dem ersten Trennbehälter, nach dem ersten Trennbehälter (und nach Zumischung der Abwässer aus der Anilin-Aufbereitung) sowie nach der TDZ angegeben.

**Tabelle 3**

| **Gehalt** | **vor erstem Trennbehälter** | **nach erstem Trennbehälter** | **nach TDZ** |
|---|---|---|---|
| Nitrobenzol | 4846 ppm | 2538 ppm | 182 ppm |
| Benzol | 357 ppm | 183 ppm | 1 ppm |
| Nitrophenolate | 13568 ppm | 13508 ppm | < 5 ppm |
| NaOH | 1,7 Gew.-% | 1,7 Gew.% | 0,9 Gew.-% |
| pH-Wert | 13,7 | 13,4 | 10,4 |
| Aminophenolate | 0 | 1241 ppm | < 5 ppm |

Die TDZ wird bei 100 bar und 290°C mit einer Verweilzeit von 30 Minuten mit einer engen Verweilzeitverteilung betrieben.

### Beispiel 4 (erfindungsgemäßes Beispiel)

Das rohe Nitrobenzol aus der adiabaten Nitrierung von Benzol wird zunächst in einer sauren Wäsche gewaschen und anschließend unter Zugabe von Natronlauge (32%ig) in einem Rührwerksbehälter alkalisch gewaschen. Danach wird das Gemisch in einem nachgeschalteten ersten Trennbehälter aufgrund der Dichteunterschiede in eine organische Phase (Rohnitrobenzol) und eine wässrige Phase (alkalisches Abwasser, Ablauge) getrennt. Das alkalische Abwasser ist mit Nitrobenzol gesättigt und liegt in der in Tabelle 4 angegebenen Zusammensetzung vor. Das alkalische Abwasser wird anschließend in einen weiteren Absetzbehälter gefördert. In diesem Absetzbehälter setzt sich nicht gelöstes Nitrobenzol und Benzol am Boden des Absetzbehälters ab und wird durch Phasentrennung abgetrennt. Danach wird die Ablauge in eine mit Direktdampf betriebene Strippkolonne eingespeist, in der Nitrobenzol und Benzol über Kopf abgestrippt wird. Das alkalische Abwasser aus dem Sumpf der Kolonne hat die in Tabelle 4 angegebene Zusammensetzung und wird zur weiteren Aufarbeitung in die thermische Druckzersetzung gefahren. Ein Teilstrom wird dabei analytisch mittels FID auf Anwesenheit von Nitrobenzol und Benzol überwacht.

**Tabelle 4**

| **Gehalt** | **vor erstem Trennbehälter** | **nach erstem Trennbehälter** | **nach Strippkolonne** | **nach TDZ** |
|---|---|---|---|---|
| Nitrobenzol | 6520 ppm | 1830 ppm | < 2 ppm | < 1 ppm |
| Benzol | 435 ppm | 155 ppm | < 1 ppm | nicht detektierbar |
| Nitrophenolate | 12250 ppm | 12150 ppm | 11850 ppm | < 5 ppm |
| NaOH | 1,0 Gew.% | 1,0 Gew.% | 0,9 Gew.-% | 0,6 Gew.-% |
| pH-Wert | 13 | 13 | 13 | 9,8 |

Die TDZ wird bei 110 bar und 275°C mit einer Verweilzeit von 30 Minuten mit einer engen Verweilzeitverteilung betrieben. Das Abwasser hat nach der TDZ die in Tab. 4 aufgeführte Zusammensetzung und kann direkt einer biologischen Kläranlage zugeführt werden.

## Patentansprüche

1. Verfahren zur Aufarbeitung von alkalischen Abwässern, die bei der Wäsche von rohem Nitrobenzol entstehen, wobei das rohe Nitrobenzol
a) durch adiabate Nitrierung von Benzol mit Nitriersäure hergestellt, und anschließend
b) in einer sauren Wäsche gewaschen, und anschließend
c) in einer alkalischen Wäsche gewaschen wird, wobei ein alkalisches Abwasser enthaltend Benzol in Konzentrationen von 100 bis 3000 ppm und Nitrobenzol in Konzentrationen von 1000 bis 10000 ppm erhalten wird,
**dadurch gekennzeichnet, dass**
d) aus dem alkalischen Abwasser aus Schritt c) anschließend nicht gelöst vorliegendes Benzol und/oder Nitrobenzol abgeschieden wird, und
e) aus dem alkalischen Abwasser aus Schritt d) anschließend restliches Benzol und/oder Nitrobenzol durch Strippen entfernt wird, und
f) das alkalische Abwasser aus Schritt d) oder e) anschließend unter Ausschluss von Sauerstoff auf Temperaturen von 150 bis 500°C unter Überdruck erhitzt wird.

2. Verfahren nach Anspruch 1, bei dem die Strippung in Schritt e) mit Wasserdampf erfolgt.

3. Verfahren nach Anspruch 1 oder 2, bei dem die Erhitzung des alkalischen Abwassers in Schritt f) bei absoluten Drücken von 50 bis 350 bar erfolgt.

4. Verfahren nach einem der Anspruche 1 bis 3, bei dem das in den Schritten d) oder e) erhaltene alkalische Abwasser mit Anilin und/oder Aminohydroxyaromaten enthaltenden Abwässern vereinigt und anschließend gemeinsam in Schritt f) erhitzt wird.

## Claims

1. Process for working up alkaline wastewater formed in the scrubbing of crude nitrobenzene, where the crude nitrobenzene is
a) prepared by adiabatic nitration of benzene by means of nitrating acid and subsequently
b) scrubbed in an acid scrub and subsequently
c) scrubbed in an alkaline scrub, giving an alkaline wastewater containing benzene in concentrations of from 100 to 3000 ppm and nitrobenzene in concentrations of from 1000 to 10 000 ppm,
**characterized in that**
d) undissolved benzene and/or nitrobenzene is subsequently separated off from the alkaline wastewater from step c) and
e) residual benzene and/or nitrobenzene is subsequently removed from the alkaline wastewater from step d) by stripping and
f) the alkaline wastewater from step d) or e) is subsequently heated to temperatures of from 150 to 500°C under superatmospheric pressure with exclusion of oxygen.

2. Process according to Claim 1, wherein the stripping in step e) is carried out by means of steam.

3. Process according to Claim 1 or 2, wherein the heating of the alkaline wastewater in step f) is carried out at absolute pressures of from 50 to 350 bar.

4. Process according to any of Claims 1 to 3, wherein the alkaline wastewater obtained in steps d) or e) is combined with wastewater containing aniline and/or aminohydroxyaromatics and subsequently heated together in step f).

## Revendications

1. Procédé pour le traitement d'eaux résiduaires alcalines, qui sont produites lors du lavage de nitrobenzène brut, le nitrobenzène brut étant
a) produit par nitration adiabatique de benzène avec du mélange sulfonitrique, et ensuite
b) lavé dans un lavage acide, et ensuite
c) lavé dans un lavage alcalin, avec obtention d'une eau résiduaire alcaline contenant du benzène à des concentrations de 100 à 3000 ppm et du nitrobenzène à des concentrations de 1000 à 10 000 ppm,
**caractérisé en ce que**
d) on sépare ensuite de l'eau résiduaire alcaline provenant de l'étape c) le nitrobenzène et/ou le benzène présent(s) non dissous, et
e) ensuite on élimine par entraînement de l'eau résiduaire alcaline provenant de l'étape d) le benzène et/ou le nitrobenzène résiduel(s), et
f) ensuite on chauffe à des températures de 150 à 500 °C sous une surpression, à l'abri de l'oxygène, l'eau résiduaire alcaline provenant de l'étape d) ou e).

2. Procédé selon la revendication 1, dans lequel l'entraînement dans l'étape e) s'effectue avec de la vapeur d'eau.

3. Procédé selon la revendication 1 ou 2, dans lequel le chauffage de l'eau résiduaire alcaline dans l'étape f) s'effectue sous des pressions absolues de 50 à 350 bars.

4. Procédé selon l'une quelconque des revendications 1 à 3, dans lequel l'eau résiduaire alcaline obtenue dans l'étape d) ou e) est épurée avec des eaux résiduaires contenant des composés aminohydroxyaromatiques et/ou de l'aniline et ensuite chauffée conjointement dans l'étape f).
